(19) Europäisches Patentamt
European Patent Office
Office européen des brevets

(11) **EP 2 380 593 A1**

(12) **EUROPEAN PATENT APPLICATION**

(43) Date of publication:
26.10.2011 Bulletin 2011/43

(51) Int Cl.:
*A61K 47/18* *(2006.01)*          *C12N 9/16* *(2006.01)*
*C12N 9/96* *(2006.01)*

(21) Application number: 11163446.5

(22) Date of filing: 21.04.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 23.04.2010 JP 2010100193

(71) Applicant: Arkray, Inc.
Minami-ku
Kyoto-shi
Kyoto 601-8045 (JP)

(72) Inventors:
• Takagi, Mikako
Kyoto-shi
Kyoto 602-0008 (JP)
• Ono, Eriko
Kyoto-shi
Kyoto 602-0008 (JP)

(74) Representative: Jones, Elizabeth Louise
Dehns
St Bride's House
10 Salisbury Square
London
EC4Y 8JD (GB)

(54) **Stabilizing labeled antibody using amino acids**

(57)    The present invention relates to a method for stabilizing a labeled antibody in a solution, in which the labeled antibody is stabilized by allowing the labeled antibody to be present together with at least one of amino acid and a derivative thereof in the solution.

EP 2 380 593 A1

**Description**

The present invention relates to a method for stabilizing a labeled antibody in a solution.

Proteins such as enzymes, labeled antibodies, and primary antibodies have been used widely in the fields of clinical testing, biochemistry, etc. However, the activities of enzymes and labeled antibodies are likely to be influenced by temperature, a chemical compound, etc., and hence, it is difficult to store an enzyme or a labeled antibody while maintaining its activity for a long period of time. Therefore, various methods for maintaining the activities of these proteins have been proposed.

An enzyme or a labeled antibody is provided as a liquid reagent in which an enzyme or a labeled antibody is dissolved in a buffer solution or a lyophilized reagent in which an enzyme or a labeled antibody is lyophilized. Various stabilization methods have been proposed for these respective forms. Regarding the liquid reagent, for example, a method for allowing crystallin to be contained in a cholesterol dehydrogenase solution (e.g., Japanese Patent No. 3,696,267) and a method for adding bovine serum albumin (BSA) to an IgM reagent solution (e.g., JP 9(1997)-127114 A) have been proposed. Further, regarding the lyophilized reagent, for example, a method for adding a saccharide such as sucrose, trehalose, or dextran to an enzyme-labeled antibody solution and lyophilizing the mixture (e.g. JP 60(1985)-149972 A), and a method for adding BSA and a saccharide or amino acid to a cholesterol oxidase solution and lyophilizing the mixture (e.g., JP 8(1996)-228774 A) have been proposed.

In the case of the lyophilized reagent, the reagent needs to be dissolved in a buffer solution, etc. for each use, which renders the operation complicated. In recent years, for ease of use, it is desireable for an enzyme or a labeled antibody to be provided as the liquid reagent. However, in the case of the liquid reagent, there are problems that a stabilizer used for stabilization is difficult to obtain and expensive. Therefore, there is a desire for a new method capable of stabilizing a labeled antibody in a liquid reagent.

The present invention provides a new method capable of stabilizing a labeled antibody in a solution.

The present invention relates to a method for stabilizing a labeled antibody in a solution, including allowing the labeled antibody to be present together with at least one of an amino acid and a derivative thereof in a solution.

According to the present invention, the labeled antibody can be stabilized in the solution.

The present invention is based on the finding that a labeled antibody can be stabilized in a solution by allowing the labeled antibody to be present together with at least one of an amino acid and a derivative thereof (hereinafter, also referred to as "amino acid") in a solution. Although the details of the mechanism, in which a labeled antibody can be stabilized in a solution by the method of the present invention, are not clear, it is presumed that, by allowing a labeled antibody to be present together with an amino acid in a solution, a decrease in activity of a marker in the labeled antibody can be suppressed, or the binding property between the marker in the labeled antibody and an antibody can be maintained. However, the present invention is not limited to any particular mechanism.

Specifically, the present invention relates to:

[1] A method for stabilizing a labeled antibody in a solution, including allowing the labeled antibody to be present together with at least one of an amino acid and a derivative thereof in the solution;

[2] The method for stabilizing the labeled antibody recited in [1], including allowing the labeled antibody to be present together with at least one of the amino acid and the derivative thereof in the solution for 24 hours or longer;

[3] The method for stabilizing the labeled antibody recited in [1] or [2], wherein the amino acid is at least one selected from the group consisting of glycine, lysine, valine, serine, and alanine;

[4] The method for stabilizing a labeled antibody according to any one of [1] to [3], wherein the labeled antibody is selected from at least one of anti-luteinizing hormone antibody, an anti-follicle-stimulating hormone antibody, an anti-human chorionic gonadotropin antibody, and an anti-thyroid-stimulating hormone antibody.

[5] The method for stabilizing the labeled antibody recited in any one of [1] to [4], wherein the labeled antibody is an enzyme-labeled antibody;

[6] The method for stabilizing the labeled antibody recited in [5], wherein the enzyme is alkaline phosphatase;

[7] A method for storing a labeled antibody as a liquid reagent, wherein the liquid reagent contains a labeled antibody and a stabilizer for stabilizing the labeled antibody in the liquid reagent, and the stabilizer is at least one of an amino acid and a derivative thereof;

[8] The method for storing the labeled antibody recited in [7], wherein the labeled antibody is stored in a solution containing the amino acid or the derivative thereof at 0°C to 15°C for 24 hours or longer;

[9] A labeled antibody liquid reagent containing a labeled antibody and a stabilizer for stabilizing the labeled antibody in a liquid reagent, wherein the stabilizer is at least one of an amino acid and a derivative thereof;

[10] A commercially available package including a labeled antibody liquid reagent containing a labeled antibody and a stabilizer for stabilizing the labeled antibody in a liquid reagent, and a container in which the labeled antibody liquid reagent is packaged, wherein the stabilizer is at least one of an amino acid and a derivative thereof;

[11] A kit comprising the labeled antibody liquid reagent recited in [9] or the commercially available package recited in [10]. The kit is suitable for assays in which a labeled antibody is used.

The term "stabilize" as used herein refers to an effect in which a labeled antibody stored for a predetermined period of time while being present together with an amino acid in a solution when compared to a labeled antibody stored for a predetermined period of time without being present together with the amino acid, has higher function remaining in the labeled antibody after storage, that is, closer to that in the state before the storage. Further, the term "stabilize" includes, for example, suppressing a decrease in function of the labeled antibody in the case where the labeled antibody is stored in the solution for a predetermined period of time.

Examples of "at least one of an amino acid and a derivative thereof" that can be used in the present invention include, but are not limited to, an amino acid and a derivative thereof (excluding polyaminoacid). Examples of the amino acid and the derivative thereof (excluding polyaminoacid) include, but are not limited to, an amino acid, and an amino acid derivative excluding polyaminoacid, preferably an a-amino acid or a salt thereof. Examples of the derivative thereof include, but are not limited to, hydroxyproline, alaninol, L-prolinol, leucinol, valinol, N-benzyloxycarbonyl-L-alanine, benzyloxycarbonyl-L-lysine, y -methyl L-glutamate, prolinamide and nitro-L-arginine. Examples of the a-amino acid include, but are not limited to, lysine, valine, serine, alanine, glycine, and glutamic acid. Lysine, valine, serine, alanine, or glycine is preferred. Examples of the salt include, but are not limited to, a sodium salt, a potassium salt, an ammonium salt, an acetate, and a hydrochloride. The polyaminoacid refers to at least two amino acids bound to each other, and examples thereof include a homopolymer such as arginine, lysine, and glutamic acid, and a random copolymer such as lysine-glycine and lysine-serine. The amino acid may be used alone or in a combination of at least two kinds.

The term "labeled antibody" as used herein refers to an antibody labeled with a marker, and for example, can be used for detecting or quantifying an antigen in a sample. Examples of the marker include, but are not limited to, an enzyme, a fluorescent material, a luminescent material, a radioisotope, biotin, and avidin. Examples of the enzyme include, but are not limited to, alkaline phosphatase, peroxidase, glucose oxidase, β-galactosidase, ß-glucosidase, malic dehydrogenase, alcohol dehydrogenase, and catalase. Examples of the fluorescent material include fluorescamine and fluorescein isothiocyanate. Other examples of the fluorescent material include, but are not limited to, luminol, a luminol derivative, and luciferin. Examples of the radioisotope include $^3$H, $^{14}$C, $^{125}$I, and $^{131}$I.

The "antibody" as used herein may be one having an antigen-binding ability, including not only an entire antibody molecule (antibody with a full length) but also an antibody fragment having the specific binding ability of an antibody or a derivative thereof. As the antibody, for example, a polyclonal antibody or a monoclonal antibody derived from the mammals or birds, or a chimera antibody, a humanized antibody or a fully human antibody that are produced artificially can be used, and specific examples thereof include, but are not limited to, IgG, IgM, IgA, IgD, and IgE. The antibody fragment refers to an antibody fragment that holds at least a part of the specific binding ability of an antibody of full length and that is shorter of full length, and examples thereof include fragments such as Fab, F(ab')2, Fab', scFv, and Fv. The antibody may comprise at least one antibody which may be employed in analytical methods associated with fertility and/or pregnancy, for example, antibodies as described hereinbefore for detecting luteinizing hormone, follicle-stimulating hormone, human chorionic gonadotropin, and/or thyroid-stimulating hormone.

The "liquid reagent" as used herein refers to a reagent in a state in which a labeled antibody is dissolved in water, a buffer solution, or the like. The liquid reagent does not require the preparation of a reagent as in a lyophilized reagent, for example, at a time of inspection, analysis, etc, and includes a reagent that can be immediately applied to inspection and analysis, a reagent that can be used as it is, etc.

[Method for stabilizing a labeled antibody]

In one aspect, the present invention relates to a method for stabilizing a labeled antibody in a solution, including allowing the labeled antibody to be present together with at least one of an amino acid and a derivative thereof in a solution. According to the stabilization method of the present invention, by allowing the labeled antibody to be present together with an amino acid in the solution, for example, the function (for example, activity) remaining in the labeled antibody after storage in the solution at 37°C for 7 days can be enhanced compared with that of a labeled antibody stored without being present together with an amino acid. Further, according to the stabilization method of the present invention, compared with the state in which the labeled antibody is not present together with an amino acid, a decrease in function (for example, activity) remaining in the labeled antibody after storage in the solution at 37°C for 7 days can be suppressed. Further, according to the stabilization method of the present invention, an amino acid that is readily available as a stabilizer of the labeled antibody is used, and hence, for example, the production process of a labeled antibody reagent is simplified and the cost thereof can be reduced.

The term "allowing a labeled antibody to be present together with amino acid in a solution" as used herein includes, for example, mixing a labeled antibody with an amino acid, and storing the solution containing the labeled antibody and the amino acid thus obtained by mixing in this state for a predetermined period of time. There is no particular limit to the mixing method of the labeled antibody and the amino acid and the order thereof. For example, after a solution of the amino acid is prepared, a labeled antibody may be added to the prepared solution, or after a labeled antibody solution is prepared, the amino acid may be added to the prepared labeled antibody solution. Alternatively, a labeled antibody and amino acid may be simultaneously added to water or a buffer solution. Although the storage time is not particularly limited, the storage time is, for example, one hour or longer, preferably 6 hours or longer, such as 24 hours or longer. Further, the storage temperature can be set appropriately depending upon the kind of labeled antibody, and is, for example, 0°C to 15°C, such as 2°C to 10°C, such as 2°C to 8°C, such as about 4°C.

The concentration of the amino acid in the solution is, for example, 0.1 to 20% by weight, such as 0.3 to 10% by weight, such as 0.5 to 5% by weight in terms of the enhancement of stabilization. Further, the molar ratio of the amino acid in the solution is, for example, 4 to 800 mol based on 1 mol of the labeled antibody, such as 12 to 400 mol, such as 20 to 200 mol based on 1 mol of the labeled antibody in terms of the enhancement of stabilization.

Examples of the solution in which a labeled antibody is present together with an amino acid include water and a buffer solution. Examples of a buffer used in the buffer solution include, but are not limited to, a Good's buffer, a phosphate buffer, and a tris buffer. Examples of the Good's buffer include, but are not limited to, N,N-bis(2-hydroxyethyl)-2-aminoethanesulfonic acid (BES), 2-morpholinoethanesulfonic acid (MES), bis(2-hydroxyethyl)iminotris(hydroxyethyl)methane(Bis-Tris), N-(2-acetamide) iminodiacetic acid (ADA), piperazine-N,N'-bis(2-ethanesulfonic acid) (PIPES), N-(2-acetamide)-2-aminoethanesulfonic acid (ACES), 3-morpholino-2-hydroxypropanesulfonic acid (MOPSO), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO), and N-cyclohexyl-3-aminopropanesulfonic acid (CAPS).

The solution may contain, for example, salts such as metal salts, saccharides, azides, BSA, and coenzymes within a range not impairing the effect of stabilization. Examples of the metal salts include, but are not limited to, sodium chloride, magnesium chloride, zinc chloride, and potassium chloride.

It is preferred that the stabilization method of the present invention does not include a lyophilization step after allowing a labeled antibody to be present together with an amino acid in a solution, and it is more preferred to store the labeled antibody and the amino acid in solution (liquid reagent) at the time of coexistence without performing a lyophilization treatment.

In one aspect of the stabilization method of the present invention, the method includes allowing an enzyme-labeled antibody to be present together with an amino acid in a buffer solution. In one embodiment, the method includes allowing the enzyme-labeled antibody to be present together with at least one amino acid selected from the group consisting of glycine, lysine, valine, serine, and alanine, in terms of the enhancement of stabilization. In another embodiment, the method includes allowing the enzyme-labeled antibody to be present together with at least one of lysine and alanine. In yet another embodiment, the method includes allowing the enzyme-labeled antibody to be present together with lysine. Thus, a decrease in activity of the enzyme-labeled antibody can be suppressed in the solution.

In one aspect of the stabilization method of the present invention, the buffer solution may contain, for example, BSA and/or sodium azide, and preferably may contain, for example, BSA, sodium azide, zinc chloride, and/or magnesium chloride.

In a preferred aspect, said stabilized labeled antibody is stored after stabilization. Thus said method provides a method of stabilizing and/or storing a labeled antibody.

[Method for storing a labeled antibody]

In another aspect, the present invention relates to a method for storing a labeled antibody as a liquid reagent, wherein the liquid reagent contains a labeled antibody and a stabilizer for stabilizing the labeled antibody in the liquid reagent, and the stabilizer is at least one of an amino acid and a derivative thereof. According to the storage method of the present invention, for example, a labeled antibody can be stored while being stabilized, and preferably can be stored for a long period of time while being stabilized. Further, according to the storage method of the present invention, compared with the state in which the labeled antibody is not present together with an amino acid, a decrease in function (for example, activity) of the labeled antibody during a storage period can be suppressed.

In the storage method of the present invention, the storage period is not particularly limited, and for example, 24 hours or longer, such as 30 days or longer, such as 12 months or longer. Further, the storage temperature can be set appropriately depending upon the kind of labeled antibody, etc., and for example, 0°C to 15°C, such as 2°C to 10°C, such as 2°C to 8°C, such as about 4°C.

In the storage method of the present invention, sealed storage in which a liquid reagent is stored while being sealed is preferred in terms of long-term storage, and more preferably, after a sealable container or the like is filled with a liquid reagent containing a labeled antibody and an amino acid, the container is sealed and stored as it is.

In the storage method of the present invention, the composition of a solution, the concentration of an amino acid in the solution, and components that can be contained in the solution are the same as those of the stabilization method of the present invention.

[Labeled antibody liquid reagent]

In another aspect, the present invention relates to a labeled antibody liquid reagent (hereinafter, also referred to as "labeled antibody reagent") containing a labeled antibody and a stabilizer for stabilizing the labeled antibody in a liquid reagent, wherein the stabilizer is at least one of an amino acid and a derivative thereof. According to the labeled antibody reagent of the present invention, for example, a labeled antibody can be stored while being stabilized. Further, according to the labeled antibody reagent of the present invention, a labeled antibody can be stored while a decrease in function (for example, activity) of the labeled antibody is suppressed, compared with the state in which the labeled antibody is not present together with an amino acid.

The concentration of the labeled antibody in the labeled antibody reagent of the present invention is, for example, 0.01 to 5 $\mu$g/mL, such as 0.05 to 2.5 $\mu$g/mL, such as 0.2 to 2 $\mu$g/mL in terms of the enhancement of stabilization.

In the labeled antibody reagent of the present invention, it is preferred that the liquid reagent contains a buffer. In the labeled antibody reagent of the present invention, the buffer, the concentration of amino acid in the solution, the other components to be contained, etc. are similar to those of the stabilization method and the storage method of the present invention.

The labeled antibody reagent of the present invention can be used as an analytical reagent for molecular biology or biochemistry, an extracorporeal diagnostic reagent used in the field of a clinical test, etc., depending upon the kind of labeled antibody to be contained, etc.

The labeled antibody reagent of the present invention can be produced by, for example, mixing a labeled antibody with at least one of amino acid and a derivative thereof in a solution.

Therefore, in another aspect, the present invention relates to a method for producing the labeled antibody reagent of the present invention, including mixing a labeled antibody with at least one of an amino acid and a derivative thereof in a solution. In the method for producing the labeled antibody reagent of the present invention, there is no particular limit to the method for mixing the labeled antibody with the amino acid and the order thereof, and for example, the labeled antibody and the amino acid can be mixed in the same way and in the same order as in the stabilization method of the present invention. Further, the method for producing the labeled antibody reagent of the present invention may include, for example, filling a container with the labeled antibody reagent obtained by mixing, and further sealing the container filled with the reagent, etc.

It is preferred that the method for producing the labeled antibody reagent of the present invention does not include a lyophilization step after mixing the labeled antibody with the amino acid in the solution.

[Still another aspect of the present invention]

In another aspect, the present invention relates to a commercially available package including a labeled antibody liquid reagent containing a labeled antibody and a stabilizer for stabilizing the labeled antibody in a liquid reagent, and a container in which the labeled antibody liquid reagent is packaged, wherein the stabilizer is at least one of an amino acid and a derivative thereof. The term "commercially available package" as used herein refers to, for example, a package in which a labeled antibody liquid reagent containing a labeled antibody and the above-mentioned stabilizer is sealed, filled, or stored in a container to be distributed or to be dealt with, and can also be referred to, for example, as a package for a product or distribution. The commercially available package of the present invention contains a labeled antibody reagent, and hence, can be used as an analytic reagent for molecular biology or biochemistry, an extracorporeal diagnostic reagent used in the field of a clinical test, etc.

In a further aspect, the present invention relates to a kit comprising the labeled antibody reagent of the present invention or the commercially available package of the present invention. The kit of the present invention contains a labeled antibody reagent, and hence, can be used as an analytic reagent for molecular biology or biochemistry, an extracorporeal diagnostic reagent used in the field of a clinical test, etc.

It is preferred that the commercially available package and the kit of the present invention further include an instruction manual describing a method for using the above-mentioned labeled antibody, etc. The commercially available package and the kit of the present invention can include the case where the manual is provided on the web without being packaged in the kit of the present invention.

Hereinafter, the present invention will be described further by way of examples and comparative examples. It should be noted that the present invention is not limited to the following examples.

Examples

<Examples 1-5, Comparative Example 1: Test for stabilization of alkaline phosphatase labeled anti-luteinizing hormone (LH) mouse monoclonal antibody>

An alkaline phosphatase labeled anti-LH mouse monoclonal antibody was used as a labeled antibody and evaluated for stability by a storage stability acceleration test.

[Labeled antibody]

The alkaline phosphatase labeled anti-LH mouse monoclonal antibody was produced by labeling an anti-LH mouse monoclonal antibody (produced by Fitzgerald Industries), using an alkaline phosphatase labeling kit (produced by Dojindo Laboratories).

[Labeled antibody solution]

The alkaline phosphatase labeled anti-LH mouse monoclonal antibody thus obtained was diluted with a reagent with the following composition to obtain an alkaline phosphatase labeled antibody solution (antibody final concentration: 0.7 μg/mL). As the stabilizer, the one shown in the following Table 1 was used.

(Composition of the reagent)

| 50 mM | Tris |
|---|---|
| 150 mM | NaC1 |
| 1 mM | $MgC1_2$ |
| 0.025 mM | $ZnC1_2$ |
| 0.1% | BSA |
| 0.05% | $NaN_3$ |
| 5% by weight | stabilizer |
| pH 7.4 | |

[Storage stability acceleration test]

The obtained alkaline phosphatase labeled antibody solution was stored in a polypropylene container at 37°C for 7 days in a light-shielded state.

[Evaluation method]

The following standard solution was added to the alkaline phosphatase labeled antibody solution before storage and allowed to react at 37°C for 5 minutes. Then, antibody sensitized beads obtained by sensitizing an anti-LH mouse monoclonal antibody were added to the reactant and allowed to react at 37°C for 5 minutes. After the reaction, the resultant reactant was washed with the following cleaning solution three times. To the washed reactant, 4-methylumbelliferyl phosphoric acid was added, and the mixture was allowed to react at 37°C for 10 minutes. After this, the fluorescent intensity (fluorescent intensity of a labeled antibody solution before storage) of 450 nm obtained by irradiating the reactant with excited light of 370 nm was measured by a fluorescent measurement apparatus.

(Composition of the standard solution)

80 mIU/mL     luteinizing hormone (LH)

(Composition of a cleaning solution)

| | |
|---|---|
| 50 mM | Tris |
| 150 mM | NaCl |
| 0.05% | Tween 20 (trade name) |
| 0.05% | $NaN_3$ |
| pH 7.4 | |

The fluorescent intensity of the labeled antibody solution after storage was measured in the same way as the above, except for using an alkaline phosphatase labeled antibody solution after storage. The residual activity (%) was calculated from the following Equation, using the fluorescent intensities of the labeled antibody solution obtained before and after storage. Table 1 shows the results.

$$\text{Residual activity } (\%) = (\text{Fluorescent intensity of labeled antibody}$$
$$\text{solution after storage})/(\text{Fluorescent intensity of labeled antibody solution}$$
$$\text{before storage}) \times 100$$

Table 1

| | Stabilizer | Residual activity (%) |
|---|---|---|
| Example 1 | lysine | 89.7 |
| Example 2 | valine | 89.1 |
| Example 3 | serine | 86.0 |
| Example 4 | alanine | 87.6 |
| Example 5 | glycine | 83.3 |
| Comparative Example 1 | None | 83.1 |

Table 1 shows that, by allowing the alkaline phosphatase labeled antibody to be present together with an amino acid in the solution, a decrease in activity of the alkaline phosphatase labeled antibody is suppressed and the alkaline phosphatase labeled antibody is stabilized. In particular, Table 1 shows that, by allowing the alkaline phosphatase labeled anti-LH mouse monoclonal antibody to be present together with lysine, valine, serine, or alanine, preferably lysine or valine, the stability of the alkaline phosphatase labeled anti-LH mouse monoclonal antibody in the solution is enhanced further.

(Reference Example)

As Reference Examples 1 and 2, a storage stability acceleration test was performed by preparing a labeled antibody solution in the same way as in the above-mentioned examples, except for using a reagent with the following composition. Table 2 shows the results. The concentration of BSA was set as shown in Table 2.

(Composition of reagent)

| 50 mM | Tris |
| 150 mM | NaCl |
| 1 mM | $MgCl_2$ |
| 0.05 mM | $ZnCl_2$ |
| 0.1 to 1% | BSA |
| 0.05% | $NaN_3$ |
| pH 7.4 | |

Table 2

| | Additive | Residual activity (%) |
|---|---|---|
| Control 1 | None (BSA 0.1%) | 76.6 |
| Reference Example 1 | BSA 0.5% | 76.0 |
| Reference Example 2 | BSA 1% | 64.5 |

As shown in Table 2, it was confirmed that, even when the concentration of the BSA is increased, the stability of the alkaline phosphatase labeled antibody in a solution state is not enhanced. Further, the residual activities after storage for 7 days with the BSA concentration of 0.5% (Reference Example 1) and 1% (Reference Example 2) decreased, compared with the case where the BSA concentration is 0.1% (control 1)

As Reference Examples 3 to 7, a storage stability acceleration test was performed by preparing a labeled antibody solution in the same way as in the above-mentioned examples, except for using a reagent with the following composition. Table 3 shows the results. The concentration of the additive (saccharide) was set as shown in Table 3.

(Composition of a reagent)

| 50 mM | Tris |
| 150 mM | NaCl |
| 1 mM | $MgCl_2$ |
| 0.05 mM | $ZnCl_2$ |
| 0.1% | BSA |
| 0.05% | $NaN_3$ |
| 0.5% by weight | stabilizer (saccharide) |
| pH 7.4 | |

Table 3

| | Additive | Residual activity (%) |
|---|---|---|
| Control 2 | None | 77.8 |
| Reference Example 3 | glucose | 77.2 |
| Reference Example 4 | sucrose | 77.1 |
| Reference Example 5 | sorbitol | 77.8 |
| Reference Example 6 | glycerin | 76.5 |
| Reference Example 7 | β-cyclodextrin | 74.8 |

As shown in Table 3, it was confirmed that the stability of the alkaline phosphatase labeled antibody is not enhanced even when the alkaline phosphatase labeled antibody is allowed to be present together with the above-mentioned saccharide in the solution.

Tables 1 to 3 show that, by allowing the alkaline phosphatase labeled antibody to be present together with an amino acid in the solution, a decrease in activity of the alkaline phosphatase labeled antibody is suppressed and the alkaline phosphatase labeled antibody is stabilized. In particular, Tables 1 to 3 show that, by allowing the alkaline phosphatase labeled antibody to be present together with lysine, valine, serine, or alanine, preferably lysine or valine, the stability of the alkaline phosphatase labeled antibody is enhanced further.

<Examples 6-10, Comparative Example 2: Test for stabilization of alkaline phosphatase labeled anti-follicle-stimulating hormone (FSH) mouse monoclonal antibody>

An alkaline phosphatase labeled anti-FSH mouse monoclonal antibody was used as a labeled antibody and evaluated for stability by a storage stability acceleration test.

[Labeled antibody]

The alkaline phosphatase labeled anti-FSH mouse monoclonal antibody was produced by labeling an anti-FSH mouse monoclonal antibody (produced by Roche Diagonistic K.K.), using an alkaline phosphatase labeling kit (produced by Dojindo Laboratories).

[Labeled antibody solution]

The alkaline phosphatase labeled anti-FSH mouse monoclonal antibody thus obtained was diluted with a reagent with the following composition to obtain an alkaline phosphatase labeled antibody solution (antibody final concentration: 0.4 μg/mL). As the stabilizer, the one shown in the following Table 4 was used.

(Composition of reagent)

| 50 mM | Tris |
| 150 mM | NaCl |
| 1 mM | $MgCl_2$ |
| 0.05 mM | $ZnCl_2$ |
| 0.1% | BSA |
| 0.05% | $NaN_3$ |
| 5% by weight | stabilizer |
| pH 7.4 | |

A storage stability acceleration test and an stability evaluation were performed in the same way as in the test for stabilization of an alkaline phosphatase labeled anti-LH mouse monoclonal antibody, except for adding the following standard solution and the alkaline phosphatase labeled antibody solution prepared as described above to antibody sensitized beads obtained by sensitizing an anti-FSH mouse monoclonal antibody, followed by allowing the mixture to react at 37 ° C for 10 minutes. Table 4 shows the results.

(Composition of the standard solution)

90 mIU/mL    follicle-stimulating hormone (FSH)

Table 4

|  | Stabilizer | Residual activity (%) |
|---|---|---|
| Example 6 | glycine | 94.0 |
| Example 7 | serine | 90.8 |
| Example 8 | lysine | 93.3 |
| Example 9 | alanine | 92.9 |
| Example 10 | valine | 91.0 |
| Comparative Example 2 | None | 88.9 |

Table 4 shows that, by allowing the alkaline phosphatase labeled antibody to be present together with an amino acid in the solution, a decrease in activity of the alkaline phosphatase labeled antibody is suppressed and the alkaline phosphatase labeled antibody is stabilized. In particular, Table 4 shows that, by allowing the alkaline phosphatase labeled anti-FSH mouse monoclonal antibody to be present together with lysine, valine, glycine, or alanine, preferably glycine, lysine or alanine, the stability of the alkaline phosphatase labeled anti-FSH mouse monoclonal antibody in the solution is enhanced further.

<Examples 11-15, Comparative Example 3: Test for stabilization of alkaline phosphatase labeled anti-human chorionic gonadotropin (hCG) mouse monoclonal antibody>

An alkaline phosphatase labeled anti-hCG mouse monoclonal antibody was used as the labeled antibody and evaluated for stability by a storage stability acceleration test.

[Labeled antibody]

The alkaline phosphatase labeled anti-hCG mouse monoclonal antibody was produced by labeling an anti-hCG mouse monoclonal antibody (produced by Medix Biochemica), using an alkaline phosphatase labeling kit (produced by Dojindo Laboratories).

[Labeled antibody solution]

The alkaline phosphatase labeled anti-hCG mouse monoclonal antibody thus obtained was diluted with a reagent with the same composition as that of Example 6 to obtain an alkaline phosphatase labeled antibody solution (antibody final concentration: 0.35 $\mu$g/mL). As the stabilizer, the one shown in the following Table 5 was used.
The alkaline phosphatase labeled antibody solution thus obtained was stored in a polypropylene container at 37°C for 7 days in a light-shielded state and the following evaluation method was performed. Table 5 shows the results.

[Evaluation method]

The following standard solution was added to antibody sensitized beads obtained by sensitizing an anti-hCG mouse monoclonal antibody and allowed to react at 37°C for 5 minutes. Then, the alkaline phosphatase labeled antibody solution before storage was added to the reactant to allow the reactant to react at 37°C for 5 minutes. After the reaction, the fluorescent intensity was measured by washing the resultant reactant in the same way as in Example 1, and the residual activity (%) of the labeled antibody was calculated based on the obtained fluorescent intensity.

(Composition of the standard solution)

250 mIU/mL    human chorionic gonadotropin (hCG)

(Composition of the cleaning solution)

| 50 mM | Tris |
| 150 mM | NaCl |
| 1 mM | $MgCl_2$ |
| 0.05 mM | $ZnCl_2$ |
| 0.1% | BSA |
| 0.05% | $NaN_3$ |
| 5% by weight | stabilizer |
| pH 7.4 | |

Table 5

| | Stabilizer | Residual activity (%) |
|---|---|---|
| Example 11 | glycine | 84.9 |
| Example 12 | serine | 83.6 |
| Example 13 | lysine | 85.9 |
| Example 14 | alanine | 82.4 |
| Example 15 | valine | 81.8 |
| Comparative Example 3 | None | 77.1 |

Table 5 shows that, by allowing the alkaline phosphatase labeled antibody to be present together with an amino acid in the solution, a decrease in activity of the antibody is suppressed and the alkaline phosphatase labeled antibody is stabilized. In particular, Table 5 shows that, by allowing the alkaline phosphatase labeled anti-hCG monoclonal antibody to be present together with glycine, serine, or lysine, preferably glycine or lysine, the stability of the alkaline phosphatase labeled anti-hCG monoclonal antibody in the solution is enhanced further.

<Examples 16-20, Comparative Example 4: Test for stabilization of alkaline phosphatase labeled anti-thyroid-stimulating hormone (TSH) mouse monoclonal antibody>

An alkaline phosphatase labeled anti-TSH mouse monoclonal antibody was used as the labeled antibody and evaluated for stability by a storage stability acceleration test.

[Labeled antibody]

The alkaline phosphatase labeled anti-TSH mouse monoclonal antibody was produced by labeling an anti-TSH mouse monoclonal antibody (produced by Fitzgerald Industries), using an alkaline phosphatase labeling kit (produced by Dojindo Laboratories).

[Labeled antibody solution]

The alkaline phosphatase labeled anti-TSH mouse monoclonal antibody thus obtained was diluted with a reagent with the same composition as that of Example 6 to obtain an alkaline phosphatase labeled antibody solution (antibody final concentration: 0.7 $\mu$g/mL). As the stabilizer, the one shown in the following Table 6 was used.
The obtained alkaline phosphatase labeled antibody solution was stored in a polypropylene container at 37°C for 7 days in a light-shielded state. The residual activity (%) of the labeled antibody in each labeled antibody solution was calculated by the same methods as those of Examples 6 to 10, except for using 50 $\mu$IU/mL of thyroid-stimulating hormone as a standard solution. Table 6 shows the results.

Table 6

| | Stabilizer | Residual activity (%) |
|---|---|---|
| Example 16 | glycine | 86.0 |

(continued)

|  | Stabilizer | Residual activity (%) |
| --- | --- | --- |
| Example 17 | serine | 87.4 |
| Example 18 | lysine | 89.2 |
| Example 19 | alanine | 88.4 |
| Example 20 | valine | 88.5 |
| Comparative Example 4 | None | 84.1 |

Table 6 shows that, by allowing the alkaline phosphatase labeled antibody to be present together with an amino acid in the solution, a decrease in activity of the alkaline phosphatase labeled antibody is suppressed and the alkaline phosphatase labeled antibody is stabilized. In particular, Table 6 shows that, by allowing the alkaline phosphatase labeled anti-TSH mouse monoclonal antibody to be present together with lysine, alanine, or valine, the stability of the alkaline phosphatase labeled anti-TSH mouse monoclonal antibody in the solution is enhanced further.
The sample analysis method of the present invention is useful for various fields such as molecular biology or biochemistry, and clinical tests.

**Claims**

1.  A method for stabilizing a labeled antibody in a solution, comprising allowing the labeled antibody to be present together with at least one of an amino acid and a derivative thereof in the solution.

2.  The method for stabilizing a labeled antibody according to claim 1, comprising allowing the labeled antibody to be present together with the at least one of an amino acid and a derivative thereof in the solution for 24 hours or longer.

3.  The method for stabilizing a labeled antibody according to claim 1 or 2, wherein the amino acid is at least one selected from the group consisting of glycine, lysine, valine, serine, and alanine.

4.  The method for stabilizing a labeled antibody according to claim any one of claims 1 to 3, wherein the labeled antibody is selected from at least one of anti-luteinizing hormone antibody, an anti-follicle-stimulating hormone antibody, an anti-human chorionic gonadotropin antibody, and an anti-thyroid-stimulating hormone antibody.

5.  The method for stabilizing a labeled antibody according to any one of claims 1 to 4, wherein the labeled antibody is an enzyme-labeled antibody.

6.  The method for stabilizing a labeled antibody according to claim 5, wherein the enzyme is alkaline phosphatase.

7.  A method for storing a labeled antibody as a liquid reagent, wherein the liquid reagent contains a labeled antibody and a stabilizer for stabilizing the labeled antibody in the liquid reagent, and
the stabilizer is at least one of an amino acid and a derivative thereof.

8.  The method for storing a labeled antibody according to claim 7, wherein the storing includes storing the labeled antibody in a solution containing the amino acid or the derivative thereof at 0°C to 15°C for 24 hours or longer.

9.  A labeled antibody liquid reagent comprising a labeled antibody and a stabilizer for stabilizing the labeled antibody in a liquid reagent, wherein the stabilizer is at least one of an amino acid and a derivative thereof.

10. A commercially available package comprising the labeled antibody liquid reagent according to claim 9 and a container in which the labeled antibody liquid reagent is packaged.

11. A kit comprising the labeled antibody liquid reagent according to claim 9 or the commercially available package according to claim 10.

Europäisches
Patentamt

European
Patent Office

Office européen
des brevets

# EUROPEAN SEARCH REPORT

Application Number

EP 11 16 3446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DAKO: "PNA ISH Detection Kit", , 14 January 2010 (2010-01-14), pages 1-22, XP002641137, Retrieved from the Internet: URL:http://www.dako.com/download.pdf?objectid=104984004 [retrieved on 2011-06-08] * page 5, paragraph 1 * | 1-11 | INV. A61K47/18 C12N9/16 C12N9/96 |
| Y | MORTON: "Some properties of alkaline phosphatase of cow's milk and calf intestinal mucosa.", BIOCHEMICAL JOURNAL, vol. 60, no. 4, 1 August 1955 (1955-08-01) , pages 573-82, XP55000455, ISSN: 0264-6021 * page 578 - page 579, left-hand column, paragraph 1 * | 1-11 | |
| Y | EP 0 709 458 A1 (DAIICHI PURE CHEMICALS CO LTD [JP]) 1 May 1996 (1996-05-01) * page 3, line 1; claims 1-3 * | 1-11 | TECHNICAL FIELDS SEARCHED (IPC) A61K C12N |
| A | "Gammagard Liquid - human immunoglobulin g injection, solution", Baxter Healthcare Corp. , September 2009 (2009-09), XP002641139, Retrieved from the Internet: URL:http://www.dailymed.nlm.nih.gov/dailymed/archives/fdaDrugInfo.cfm?archiveid=26352 [retrieved on 2011-06-08] * the whole document * | 1-11 | |

-/--

The present search report has been drawn up for all claims

| Place of search | Date of completion of the search | Examiner |
|---|---|---|
| Munich | 27 June 2011 | Lechner, Oskar |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

EUROPEAN SEARCH REPORT

Application Number

EP 11 16 3446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GB 1 120 298 A (BIOREX LABORATORIES LTD) 17 July 1968 (1968-07-17) * the whole document * ----- | 1-11 | |
| A | GB 900 115 A (ARMOUR & CO) 4 July 1962 (1962-07-04) * the whole document * ----- | 1-11 | |
| A | GB 824 290 A (ORTHO PHARMA CORP) 25 November 1959 (1959-11-25) * the whole document * ----- | 1 | |
| | | | TECHNICAL FIELDS SEARCHED (IPC) |

The present search report has been drawn up for all claims

| Place of search | Date of completion of the search | Examiner |
|---|---|---|
| Munich | 27 June 2011 | Lechner, Oskar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 16 3446

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2011

| Patent document cited in search report | | Publication date | Patent family member(s) | | Publication date |
|---|---|---|---|---|---|
| EP 0709458 | A1 | 01-05-1996 | DE | 69502905 D1 | 16-07-1998 |
| | | | DE | 69502905 T2 | 08-10-1998 |
| | | | DE | 69532334 D1 | 29-01-2004 |
| | | | DE | 69532334 T2 | 03-06-2004 |
| GB 1120298 | A | 17-07-1968 | NONE | | |
| GB 900115 | A | 04-07-1962 | NONE | | |
| GB 824290 | A | 25-11-1959 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3696267 B
- JP 9127114 A
- JP 60149972 A
- JP 8228774 A